# EUROPÄISCHE PATENTSCHRIFT

(11) **EP 3 585 474 B1**
(45) Veröffentlichungstag und Bekanntmachung des Hinweises auf die Patenterteilung: **15.05.2024**
(21) Anmeldenummer: 18705416.8
(22) Anmeldetag: 21.02.2018
(51) Int. Cl.: A61M 27/00, A61B 5/00, A61B 5/03, A61B 5/0205, A61B 5/1455

(54) **VORRICHTUNG FÜR EINE GEHIRNDRAINAGE**
DEVICE FOR DRAINAGE OF THE BRAIN
DISPOSITIF POUR PRATIQUER UN DRAINAGE CÉRÉBRAL

(30) Priorität: 24.02.2017 EP 17157971
(43) Veröffentlichungstag der Anmeldung: 01.01.2020
(73) Patentinhaber: SNP - Smart Neuro Products GmbH, 70619 Stuttgart (DE)
(72) Erfinder:
(74) Vertreter: Maiwald GmbH
(86) Internationale Anmeldenummer: PCT/EP2018/054316
(87) Internationale Veröffentlichungsnummer: WO 2018/153943

(56) Entgegenhaltungen:
- EP-A2- 0 589 862
- EP-A2- 1 702 641
- US-A1- 2012 302 938
- US-A1- 2014 276 660
- US-A1- 2016 287 111

## Beschreibung

### Technische Gebiet

Die Erfindung betrifft eine Gehirndrainagevorrichtung, insbesondere eine Gehirndrainagevorrichtung mit einer integrierten Sensoranordnung zur Überwachung von physikalischen Parametern, insbesondere des Gehirndrucks während eines Eingriffs.

### Hintergrund der Erfindung

Der Hirndruck ist ein umgangssprachlicher Begriff für den Druck, der im Schädelinneren herrscht (intrakranieller Druck, häufige, auch im Medizinischen verwendete Abkürzung ICP für englisch intracranial pressure). Der intrakranielle Druck ist entscheidend für die Durchblutung und somit für die Funktion des Gehirns, da er dem Druck, mit dem das Blut ins Gehirn gepumpt wird, entgegenwirkt. Sind intrakranieller Druck und mittlerer arterieller Blutdruck gleich, wird das Gehirn nicht mehr durchblutet. Das Gehirn reduziert in kurzer Zeit seine Funktionstätigkeit und stirbt innerhalb kurzer Zeit ab. Daher ist die Messung des intrakraniellen Drucks ein wichtiger Anhaltspunkt für therapeutische Entscheidungen bei der Behandlung von Patienten mit schweren Hirnschädigungen, z.B. nach einem Schädel-Hirn-Trauma oder einem Schlaganfall.

Messungen des intrakraniellen Drucks kommen z.B. zum Einsatz im Bereich der raumfordernden post-operativen Tumor-Nachsorge, bei Schädel-Hirn Traumata, parenchymatösen und intraventrikulären Hirnblutungen und Schlaganfall. Erhöhter intrakranieller Druck kann z.B. zu Stauungspapillen, Kopfschmerz und Erbrechen führen.

Konventionelle Messmethoden des intrakraniellen Drucks sind entweder nichtinvasiv und bieten damit einen gewissen Patientenkomfort mit einhergehender Messgenauigkeit oder invasiv bei höherer Messgenauigkeit aber deutlich geringerem Patientenkomfort.

Der Liquor cerebrospinalis (Gehim-Rückenmarks-Flüssigkeit, Zerebrospinalflüssigkeit, CSF) ist eine klare und farblose Körperflüssigkeit, die mit der Gewebsflüssigkeit des Gehirns in Verbindung steht und daher auch in der Zusammensetzung sehr ähnlich ist. Der Liquor wird in den Plexus choroidei hauptsächlich in den Seitenventrikeln (aber auch in Teilen des dritten und vierten Ventrikels) aus dem Blut abgepresst und gelangt über den dritten Ventrikel und den Aquaeductus mesencephali in den vierten Ventrikel und von dort durch Öffnungen (Aperturae) in den äußeren Liquorraum außerhalb des Gehirns. Hier findet die Wiederaufnahme (Resorption) des Hirnwassers in venöse Blutleiter statt. Es handelt sich also um ein zirkulierendes System.

Ist die Liquorproduktion zu stark oder kann der Liquor nicht ordnungsgemäß abfließen, weil ein Tumor, eine Hirnblutung oder eine andere Schwellung den Abfluss versperrt, dann entsteht ein Hydrocephalus internus, eine Stauung des Hirnwassers im Ventrikelsystem. Diese kann das Gehirn unter Druck setzen (Hirndruck), der lebensgefährlich sein kann. Um diese Lebensgefahr abzuwenden, muss der Druck aus dem Ventrikel abgeleitet werden. Daher wird von außen in einer kleinen Operation, die entweder im Operationssaal oder bettseitig auf der Intensivstation erfolgen kann, ein Schlauch oder eine dünne Nadel (Duisburger Nadel) durch die Schädeldecke in das Ventrikelsystem eingeführt. Durch diese Ableitung kann dann der Liquor entweichen und die Gefahr ist zunächst gebannt.

Da eine solche externe Ventrikeldrainage eine potenzielle Eintrittspforte für Keime ist, kann sie nicht unbegrenzt liegen bleiben. Sie ist also nur als vorübergehende Ableitung geeignet. Ist eine dauerhafte Liquorableitung erforderlich, muss operativ ein sogenannter interner Cerebralshunt z.B. in Form eines Ventrikulo-peritonealen Shunts angelegt werden.

US 2016/0287111 A1 offenbart eine drahtlose Druckmessung und Überwachung für Hirnwassershunts. EP 0 589 862 A2 beschreibt eine Sensoranordnung mit einem in ein Gewebe einführbaren Röhrchen. Die Messung kann mit einem Lichtleiter mittels Totalreflexion geführt werden.

### Zusammenfassung der Erfindung

Durch die vorliegende Erfindung wird eine verbesserte Gehirndrainagevorrichtung gemäß den unabhängigen Ansprüchen bereitgestellt, insbesondere eine Gehirndrainagevorrichtung mit einer integrierten Sensoranordnung, insbesondere zur Hirndruckmessung. Vorteilhafte Ausführungsformen und Weiterbildungen sind in den abhängigen Ansprüchen und der nachfolgenden Beschreibung verkörpert.

Gemäß einer Ausführungsform der Erfindung wird eine Gehirndrainagevorrichtung vorgeschlagen mit einem stabförmigen Hohlkörper mit einem inneren Drainagekanal zur Einführung durch die Schädeldecke in das Gehirn, einer ersten Sensoranordnung mit wenigsten einem Sensor zur Messung eines physikalischen Parameters und einer Signalschnittstelle; wobei der stabförmige Hohlkörper einen ersten Bereich aufweist, der ausgelegt ist, um im applizierten Zustand in den im Gehirn befindlichen Ventrikel hineinzuragen; wobei der stabförmige Hohlkörper einen zweiten Bereich aufweist, der proximal von dem ersten Bereich angeordnet ist, wobei der zweite Bereich ausgelegt ist, um im applizierten Zustand im Bereich der Gehirnmasse zu liegen; wobei der erste Bereich eine mit dem Drainagekanal verbundene Drainageöffnungsanordnung aufweist, über die im applizierten Zustand Liquor aus dem Ventrikel in den Drainagekanal drainagiert werden kann; wobei die erste Sensoranordnung im zweiten Bereich angeordnet ist, um einen physikalischen Parameter der Hirnmasse zu messen; wobei die erste Sensoranordnung mit der Signalschnittstelle verbunden ist, um von der ersten Sensoranordnung ermittelte Messdaten an ein zu verbindendes Messsystem zu übermitteln, wobei die erste Sensoranordnung wenigstens einen Sensor zur Erfassung eines Sauerstoffgehaltes und/oder einer Sauerstoffsättigung im Bereich der Gehirnmasse oder des Ventrikels aufweist, wobei der Sensor wenigstens eine Lichtquelle für Licht mit wenigstens einer ersten Wellenlänge und einer zweiten Wellenlänge aufweist, sowie wenigstens einen der Lichtquelle zugeordneten und bezüglich der erste Wellenlänge sensitiven ersten Lichtsensor und einen der Lichtquelle zugeordneten und bezüglich der zweite Wellenlänge sensitiven zweiten Lichtsensor.

Auf diese Weise kann eine schonende und zuverlässige Drainagierung des Ventrikels vorgenommen werden und gleichzeitig die relevanten physikalischen Parameter überwacht werden, die Ausdruck des medizinischen Zustandes des Patienten sind.

In einer Ausführungsform der Erfindung weist die erste Sensoranordnung im Wesentlichen eine geometriekonstante Form auf. Mit anderen Worten, die Sensoranordnung und der Messvorgang hat keinen Einfluss auf die Geometrie der Gehirndrainagevorrichtung. Auf diese Weise kann eine Auswirkung der Sensoren und der Gehirndrainagevorrichtung auf den Organismus des Patienten reduziert werden.

Gemäß einer Ausführungsform der Erfindung kann die Gehirndrainagevorrichtung mehrere Sensoren aufweisen, die mit einem anzuschließenden Messsystem verbunden sind. Die mehreren Sensoren können beispielsweise unterschiedliche physikalische Parameter messen. Die Sensoren können zusammen in einer Baueinheit gefasst sein und so fusioniert sein. Beispielsweise kann eine Fusion von Sensoren für die Temperatur, Hirndruckmessung und/oder Hirnaktivität in einer Sensoranordnung vorgenommen werden. Die Sensoranordnung kann außen auf den Hohlkörper aufgebracht werden. Möglich ist die Aufbringung der Sensoranordnung zur Messung von z.B. Hirndruck, Temperatur und/oder Gehirnaktivitätsmessung (EEG) mit einem Druckverfahren, etwa einem galvanischen, lithographischen oder Jet-Druckverfahren. Die Sensoranordnung kann auch innen durch den Hohlkörper verlaufen. Die Sensoranordnung kann mehrere Sensoren gleicher oder verschiedener Art umfassen. Die Sensoren können an unterschiedlichen Positionen und/oder mit unterschiedlichen Messwinkeln versehen sein. Die Sensoren können in Vertiefungen an der Außenseite des stabförmigen Hohlkörpers eingelassen sein, sodass diese flächenbündig mit der Außenseite des Hohlkörpers fluchten und nicht auftragen.

Gemäß einer Ausführungsform der Erfindung kann der Hohlkörper der Gehirndrainagevorrichtung aus einer Titanlegierung oder Titan sein. Der Hohlkörper, der die Messsensorik aufnimmt, kann aus einem nicht-magnetischen, strahlungsdurchlässigem Material bestehen. Das Material kann metallisch, insbesondere Titan oder eine Titanlegierung oder Kunststoff sein. Die Vorteile in einem metallischen Material werden darin gesehen, dass die Länge im Vergleich zu Kunststoff besser kalibrierbar ist. Der Hohlkörper kann mit einem Kunststoff überzogen sein. Der Hohlkörper kann sterilisierbar sein und kann insbesondere durch Hitze sterilisierbar sein.

Gemäß einer Ausführungsform kann der Hohlkörper z.B. einen Mindestdurchmesser von 1 mm haben, der Maximaldurchmesser kann z.B. bei 10 mm liegen.

Gemäß einer Ausführungsform kann die Gehirndrainagevorrichtung mehrere oder nur eine Drainageöffnung aufweisen. Der stabförmige Hohlkörper kann gemäß einer Ausführungsform die Drainageöffnungen ausschließlich an den zylindrischen Teilen aufweisen, sodass der Stirnbereich ohne Öffnung ausgestaltet ist. Dies verringert die Verletzungsgefahr beim Einführen des stabförmigen Hohlkörpers in den Schädel bzw. die Gehirnmasse des Patienten. Alternativ kann eine einzelne Drainageöffnung eine Öffnung einer entlang der Längsachse des Hohlkörpers verlaufenden Durchgangsbohrung am Ende des Hohlkörpers sein. Die Drainageöffnungen können dabei oval oder elliptisch ausgestaltet sein und an den Kanten zu der Außenseite des Hohlkörpers gerundet sein um Verletzungen zu vermeiden.

Gemäß einer Ausführungsform der Erfindung kann die Gehirndrainagevorrichtung in dem ersten, mit der Drainageöffnungsanordnung versehenen Bereich eine zweite Sensoranordnung aufweisen mit wenigstens einem Sensor aus der Gruppe bestehend aus: Sauerstoffgehaltsensor, Sauerstoffsättigungssensor, Drucksensor und Temperatursensor, um einen entsprechenden physikalischen Parameter auch im Ventrikel zu ermitteln. Die zweite Sensoranordnung kann auch mit der Signalschnittstelle verbunden sein, um von der zweiten Sensoranordnung ermittelte Messdaten an ein zu verbindendes Messsystem zu übermitteln. Auf diese Weise können auch im Ventrikelbereich physikalische Parameter ermittelt und ausgelesen werden.

Gemäß einer Ausführungsform der Erfindung weist wenigstens ein Sensor der zweiten Sensoranordnung einen Sensor zur Erfassung eines Sauerstoffgehaltes im Bereich der Gehirnmasse oder des Ventrikels auf mit wenigstens einer Lichtquelle für Licht mit wenigstens einer ersten Wellenlänge und einer zweiten Wellenlänge, sowie wenigstens einen der Lichtquelle zugeordneten und bezüglich der erste Wellenlänge sensitiven ersten Lichtsensor und einen der Lichtquelle zugeordneten und bezüglich der zweite Wellenlänge sensitiven zweiten Lichtsensor. Der erste und der zweite Lichtsensor können auch fusioniert in einer Baueinheit vorgesehen sein. Gemäß einer Ausführungsform können die Lichtquelle und die zugeordneten Lichtsensoren axial und/oder azimutal beabstandet zueinander angeordnet sein.

Auf diese Weise kann die Sauerstoffsättigung auf der Grundlage von zwei unterschiedlichen Lichtwellenlängen ermittelt werden, die beispielsweise im Bereich des roten bzw. infraroten Lichtes liegen, d.h. z.B. bei einer Wellenlänge von größer als 570nm oder 600nm.

Gemäß einer Ausführungsform der Erfindung weist die Gehirndrainagevorrichtung im Drainagekanal proximal der Drainageöffnungsanordnung eine steuerbare Ventilanordnung auf, um den Drainagekanal gezielt öffnen und schließen zu können. Dies kann entweder durch einen am Ventil einstellbaren Schwelldruck erfolgen, oder in Abhängigkeit von einem durch eine der Sensoranordnungen gemessenen Druck. Das Messsystem kann dazu einen einstellbaren Regelkreis aufweisen, über den die Ventilanordnung automatisch angesteuert werden kann, um einen voreingestellten Druck aufrecht zu erhalten.

Gemäß einer Ausführungsform weist die steuerbare Ventilanordnung einen mit einer Innenwandung des Drainagekanals verbundenen Ventilsitz und ein dazu in Bezug zu dem Drainagekanal verschiebbares und gegenüber dem Ventilsitz abdichtendes Ventilelement auf. Dieses kann beispielsweise durch ein Piezoelement bewegt werden. Das Ventilelement kann strömungstechnisch optimiert sein und beispielsweise eine Ellipsoidform oder Projektilform aufweisen.

Gemäß einer Ausführungsform der Erfindung kann ein Abschnitt der Innenwandung des Drainagekanals, der Ventilsitz und das Ventilelement als eine vormontierte bzw. vorgefertigte Einheit ausgebildet sein, die bei der Fertigung der Gehirndrainagevorrichtung in den stabförmigen Hohlkörper eingebracht wird. Dazu kann im stabförmigen Hohlkörper im Drainagekanal ein Absatz bzw. Rücksprung vorgesehen sein, sodass die vorgefertigte Einheit im montierten Zustand eine zusammen mit dem Grundkörper des stabförmigen Hohlkörpers eine fluchtende Innenwand des Drainagekanals bildet.

Gemäß einer Ausführungsform der Erfindung kann der stabförmige Hohlkörper Markierungen für z.B. die Eindringtiefe aufweisen, die beispielsweise durch einen Laser oder ein Ätzverfahren sehr präzise aufgebracht werden können. Der Hohlkörper kann an seinem freien distalen Ende eine gewölbte Oberfläche aufweisen, um Hirnmasse beiseite zu drücken.

Gemäß einer Ausführungsform der Erfindung kann der distale Bereich des stabförmigen Hohlkörpers eine Verdickung aufweisen, hergestellt z.B. dadurch, dass eine Kugel mit einer Durchgangsbohrung auf den stabförmigen Hohlkörper geschoben und mit diesem fest und lückenfrei verbunden wird. Auf diese Weise kann die Gehirnmasse beim Durchschieben der Gehirndrainagevorrichtung sanft beiseite gedrückt werden.

Unter distal wird ein Bereich in Richtung des menschlichen Körpers bzw. der Körpermitte verstanden, hier dem Bereich mit Drainageöffnungen. Unter proximal wird ein Bereich in vom menschlichen Körper weg gerichteter Richtung verstanden.

Als magnetisch inaktives Material wird Material verstanden, welches sich bei magnetfeldbasierten Untersuchungsverfahren im Wesentlichen neutral verhält und keine signifikanten magnetischen Eigenschaften entwickelt. Dadurch sollen bei bildgebenden Verfahren, z.B. MRT, störende Artefakte vermieden werden. Derartige Materialien können z.B. Titan bzw. Titanlegierungen oder Kunststoffe sein. Titan hat den Vorteil, dass es sich thermisch sterilisieren lässt. Kunststoffe sind leicht zu verarbeiten.

Als geometriekonstante Form wird eine Form verstanden, die ihre geometrische Form nicht signifikant verändert. Ein Drucksensor mit einer Druckmembran verändert die Form der Gehirndrainagevorrichtung nicht signifikant. Ein Ballonkatheter hingegen, insbesondere, wenn er zu Druckmessungen herangezogen wird, verändert seine Form und ist raumergreifend. Für Anwendungen im Gehirn, insbesondere bei erhöhten Gehirndrücken, ist das jedoch nicht immer erwünscht, da es zu unvorhersehbaren Druckbelastungen kommen kann.

Die Festlegung an der Schädeldecke kann direkt oder indirekt erfolgen, etwa durch eine Schneideschraube, in der der stabförmige Hohlkörper festgelegt ist oder festlegbar ist.

Drucktechnisch hergestellte Signalverbindung oder auch Sensoren können z.B. gedruckte Leiterbahnen oder Elektroden und Isolatorschichten sein. Diese könne auch in Multilayertechnik hergestellt sein. Dazu zählen auch Herstellungsverfahren wie eine galvanische Abscheidung, chemisches Verändern und/oder Ätztechniken wie in der Halbleiterherstellung verwendet, Lithographieverfahren oder JetVerfahren analog zu einem Tintenstrahldrucker. Die Leiterbahnen sind üblicherweise gegeneinander und nach außen mit einer isolierenden Schicht versehen. Die äußere isolierende Schicht kann auch durch das Coating des gesamten stabförmigen Hohlkörpers erfolgen. Dieses Coating kann z.B. durch eine Pulverbeschichtung erfolgen. Isolationsschichten, sowohl für die Leiterbahnen, als auch für die Sensoren, etwa zur Isolierung gegenüber dem stabförmigen Hohlkörper, können auch mittels Sputtertechnik aufgebracht werden, etwa durch Sputtern von Glas, welches so spezifiziert ist, dass es weder splittert noch bricht.

### Kurze Beschreibung der Figuren

Die Erfindung wird anhand folgender Figuren erläutert und verdeutlicht:
- Fig. 1: zeigt eine Schnittansicht einer erfindungsgemäßen Anordnung (nicht maßstabsgerecht).
- Fig. 2: zeigt eine mögliche Applikation der erfindungsgemäßen Anordnung an der menschlichen Anatomie.
- Fig. 3: zeigt eine gestufte bzw. abgesetzte Geometrie des stabförmigen Hohlkörpers gemäß einer Ausführungsform der Erfindung.
- Fig. 4: zeigt eine konisch nach distal zulaufende Geometrie des stabförmigen Hohlkörpers gemäß einer Ausführungsform der Erfindung.
- Fig. 5: zeigt eine Ausgestaltung des Spitzenbereiches bzw. Ventrikelbereiches gemäß einer Ausführungsform der Erfindung.
- Fig. 6: zeigt eine beispielhafte Anordnung mehrerer gleichartiger Sensoren, um z.B. eine ortsaufgelöste Messung vornehmen zu können.
- Fig. 7: zeigt eine Anordnung einer Ventilanordnung im Drainagekanal zur Drucksteuerung im Ventrikel.
- Fig. 8: zeigt eine beispielhafte Anordnung eines Sauerstoffmess- /Sauerstoffsättigungssensors mit einer oder mehreren Lichtquellen und auf gleicher Längenposition angeordneten Lichtsensoren.
- Fig. 9: zeigt eine Schnittansicht der Anordnung der Fig. 8 mit Schnittverlauf durch den Sensorkranz.
- Fig. 10: zeigt eine beispielhafte Anordnung eines Sauerstoffmess- /Sauerstoffsättigungssensors mit einer oder mehreren Lichtquellen und auf beabstandeten Längenpositionen angeordneten Lichtsensoren.
- Fig. 11: zeigt eine Schnittansicht der Anordnung der Fig. 10 mit Schnittverlauf durch den Sensorkranz, bei dem in jedem Kranz im Wesentlichen nur eine Art von Sensor/Lichtquelle angeordnet sind.

Weitere Merkmale, Vorteile und Anwendungsmöglichkeiten der vorliegenden Erfindung ergeben sich aus der nachfolgenden Beschreibung der Ausführungsbeispiele und den Figuren. In den Figuren stehen gleiche Bezugszeichen für gleiche oder ähnliche Objekte.

### Detaillierte Beschreibung beispielhafter Ausführungsformen

Die vorliegende Erfindung sieht eine Messung unmittelbar an einer Sonde einer Gehirndrainagevorrichtung vor. Darüber hinaus sieht die Erfindung unter anderem vor mehrere Messverfahren (z.B. ICP (intrakraniale Druckmessung) über eine Sonde, Druckmessung der zerebralen Durchblutung und Messung der Hirnaktivität, der Temperatur etc.) miteinander zu verbinden und die ICP, Durchblutungsmessung und Hirnaktivität in einem Gerät zu vereinen. Konventionell finden solche Messverfahren unabhängig voneinander statt und es gibt keine aggregierte Datenbasis aus diesen Messungen. Weiterhin leiden bekannte Messverfahren daran, dass die Ungenauigkeit der Druckmessung zunimmt, je höher der ICP wird.

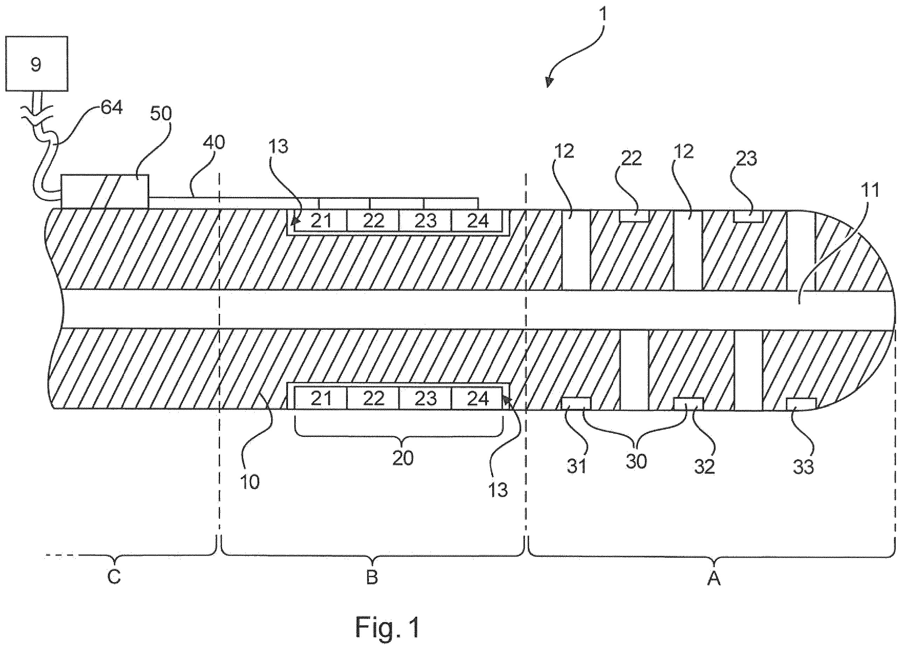

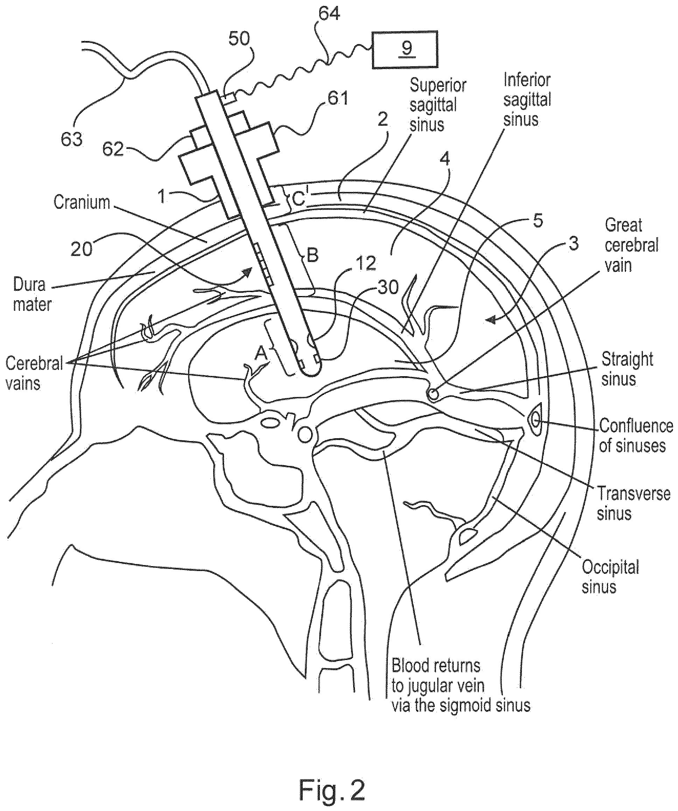

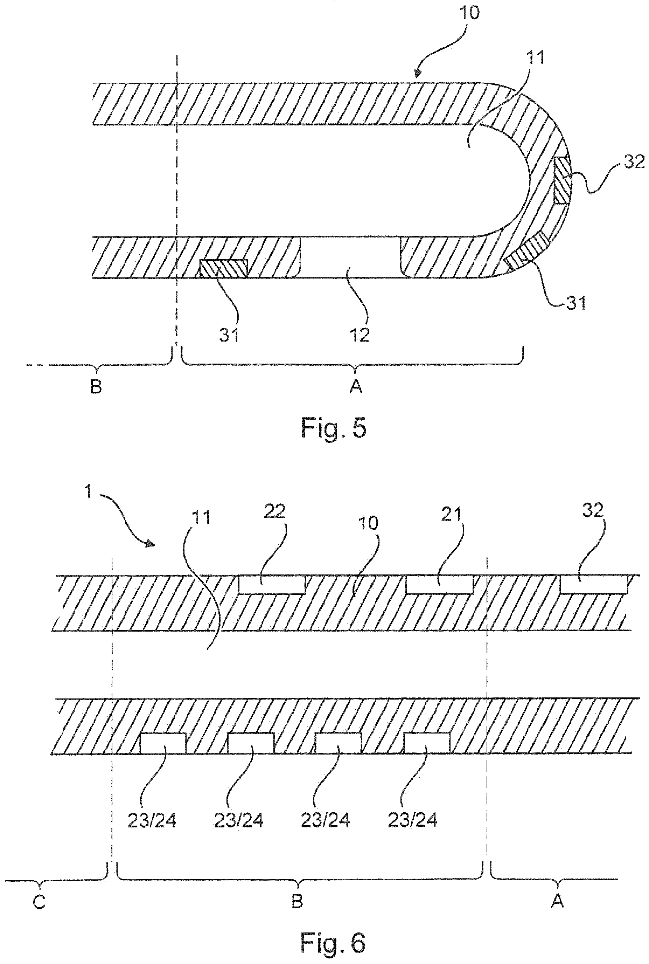

Figur 1 zeigt eine beispielhafte Ausführungsform der Erfindung, wobei die Gehirndrainagevorrichtung 1 in einer Querschnittsansicht gezeigt ist. Die Gehirndrainagevorrichtung weist beispielsweise drei Bereiche A, B, C auf. Wie in Figur 2 zu sehen ist, liegt der Bereich A im applizierten Zustand im Ventrikel 5 des Gehirns. Der Bereich B liegt im Bereich der Gehirnmasse, während der Bereich C im Bereich der Schädeldecke oder außerhalb des Schädels liegt. Die Gehirndrainagevorrichtung 1 weist einen stabförmigen Hohlkörper 10 auf, in dessen Inneren ein innerer Drainagekanal vorgesehen ist. In dem stabförmigen Hohlkörper 10 sind Drainageöffnungen 12 vorgesehen, die den Drainagekanal 11 mit der Außenseite des stabförmigen Hohlkörpers 10 verbinden. Die Drainageöffnungen 12 können dabei entweder nur an dem zylindrischen Teil des Bereiches A vorgesehen sein, sodass die Stirnseite im Wesentlichen integral geschlossen bleibt, so wie in Figur 5 gezeigt, oder auch an der Stirnseite vorgesehen sein, so wie in Figur 1 gezeigt. Im Bereich B, der im Bereich der Gehirnmasse liegt, kann eine Sensoranordnung 20 vorgesehen sein, die wiederum mehrere unterschiedliche Einzelsensoren aufweisen kann, die jedoch zu der Sensoranordnung 20 fusioniert sein können. Die Sensoren 21, 22, 23, 24 können dabei Drucksensoren, Temperatursensoren, Gehirnaktivitätssensoren sein und eine erste Sensoranordnung weist wenigstens einen Sensor zur Erfassung eines Sauerstoffgehaltes und/ oder einer Sauerstoffsättigung. Die Sensoren bzw. die Sensoranordnung 20 kann dabei in eine entsprechende Ausnehmung 13 an der Außenseite des stabförmigen Hohlkörpers 10 eingelassen sein, sodass die Sensoren 20 nicht über die Außenseite des stabförmigen Hohlkörpers 10 hinausragen. Auf diese Weise kann eine integrale Oberfläche geschaffen werden, die das Risiko von Verletzungen oder ungewünschte Verformungen beim Applizieren reduzieren kann. Der Zwischenraum zwischen den Sensoren 21, 22, 23, 24 und dem stabförmigen Hohlkörper kann mit einer Isolationsschicht versehen sein. Diese kann beispielsweise ein polymerer Isolierstoff oder auch Glas sein. Die Sensoren können im eingelassenen Zustand auch mit einer Beschichtung versehen sein, die sich auch über die verbleibende Außenseite des stabförmigen Hohlkörpers 10 erstrecken kann, um Spalten zu verschließen. Die Sensoren können mittels einer Signalverbindung 40 mit einer Signalschnittstelle 50 verbunden sein, wobei die Signalschnittstelle im Wesentlichen außerhalb des eigentlichen Schädelbereiches liegt, hier im Bereich C. Die Verbindung zwischen den Sensoren und der Signalschnittstelle 50 kann durch eine aufgedruckte Signalleitung erfolgen, die beispielsweise durch ein lithographisches Verfahren, ein Jetverfahren ähnlich eines Tintenstrahldruckers oder auch eines galvanischen Verfahrens hergestellt werden kann. Derartige Signalleitungen 40 können auch in einer Multilayertechnik hergestellt werden. Die Signalleitungen sollen jedenfalls so vorgesehen sein, dass sie im Wesentlichen nicht auftragen und die äußere Geometrie des stabförmigen Hohlkörpers bzw. der Gehirndrainagevorrichtung 1 im Wesentlichen nicht beeinträchtigen. Über die Signalschnittstelle 50 können die Signale dann über eine entsprechende Signalleitung 64 oder auch drahtlos an eine Messanordnung bzw. ein Messsystem weitergegeben werden. Die Energieversorgung der Sensorik kann drahtgebunden oder auch drahtlos erfolgen, z.B. über eine induktive Kopplung. Alternativ kann die notwendige Energie auch über eine Temperaturdifferenz des Körpers zur Umgebung gewonnen werden. Zu diesem Zweck kann gemäß einer Ausführungsform die Gehirndrainagevorrichtung ein Thermoelement aufweisen. Sofern für die drainagierte Flüssigkeit ein Sammelbehälter vorgesehen ist und die Signalübertragung über eine Drahtlosübertragung erfolgt, kann die gesamte Gehirndrainagevorrichtung ohne störende Zu- und Ableitungen vorgesehen sein, was die Anwendung wesentlich komfortabler macht.

Im Bereich A, der bei der Applikation in einem Ventrikel des Gehirns liegt, können ferner weitere Sensoren 31, 32, 33 vorgesehen sein, die auch Teil einer zweiten Sensoranordnung 30 sein können. Diese Sensoren 31, 32, 33 können beispielsweise Temperatursensoren, Drucksensoren oder Sauerstoffsättigungssensoren sein. Diese Sensoren können, obwohl dies in Figur 1 nicht dargestellt ist, ebenfalls mit entsprechenden Signalleitungen an die Signalschnittstelle 50 angebunden sein.

Die Messung des Hirninnendrucks, der zerebralen Durchblutung und der neuronalen Aktivitäten soll nach dem in Figur 2 gezeigten Schema erfolgen. Die Sonde weist eine äußere Drainageführung, auf die in axialer Länge bis in die Ventrikel des Gehirns hineinreicht und dem Abtransport überschüssiger Liquor Flüssigkeit dient. Richtung Schädeldecke ist diese Drainage gegen Herausfallen gesichert durch eine Verschraubung. Eine zusätzliche Sicherung ist durch eine Sicherung, z.B. eine Kontermutter gegeben. Im Bereich A und B befindet sich z.B. die Messeinrichtung für Druck, Temperatur, Sauerstoffsättigung und Hirnaktivität. Diese Signale werden über ein Kabel 64 nach außen geleitet und an das externe Messgerät angeschlossen. Dabei kann auch eine Datenübertragung an ein externes Messgerät über RFID, WLAN oder andere drahtlose Verfahren erfolgen.

Figur 2 zeigt nun die Applikation der Gehirndrainagevorrichtung 1 an einem menschlichen Schädel. Im eingebrachten und applizierten Zustand liegt der Bereich A im Bereich des Ventrikels 5, sodass die Drainageöffnungen 12 im Bereich des Ventrikels liegen, um eine dort vorhandene Flüssigkeit drainagieren zu können. Wie in Figur 2 zu sehen ist, liegen die Sensoren der ersten Sensoranordnung 20 im Bereich B, der im applizierten Zustand im Bereich der Gehirnmasse liegt. In diesem Bereich können die Temperatur, der Druck, eine Sauerstoffsättigung bzw. eine Gehirnaktivität gemessen werden. Die Sensoren der zweiten Sensoranordnung 30 liegen im Bereich des Ventrikels, um auch an dieser Stelle physikalische Parameter aufnehmen zu können. Über in Figur 2 nicht dargestellte Signalverbindungen können die erfassten Messsignale an eine Signalschnittstelle 50 weitergeleitet werden, um dann über eine Signal- bzw. Datenleitung 64 an das Messsystem weitergeleitet zu werden. Es sei verstanden, dass das Messsystem auch integral an der Gehirndrainagevorrichtung 1 vorgesehen sein kann, sodass keine explizite Signal- bzw. Datenleitung 64 notwendig ist.

Im Bereich C, der im Bereich der Schädeldecke liegt, kann die Gehirndrainagevorrichtung 1 an der Schädeldecke 2 festgelegt werden. Auf diese Weise kann vermieden werden, dass sich die Gehirndrainagevorrichtung 1 in Bezug zum Schädel verschiebt und sich damit auch die Sensoranordnungen verschieben, die möglicherweise infolgedessen Messfehler generieren könnten. Der stabförmige Hohlkörper 10 bzw. die Gehirndrainagevorrichtung 1 können mittels eines Cranialblocks an der Schädeldecke 2 festgelegt werden, wobei der Cranialblock 61 mit einer Kontermutter 62 an dem stabförmigen Hohlkörper 10 verriegelt werden kann. Darüber hinaus kann die Gehirndrainagevorrichtung 1 eine Drainageleitung 63 aufweisen, über die drainagierte Flüssigkeit abgeführt werden kann.

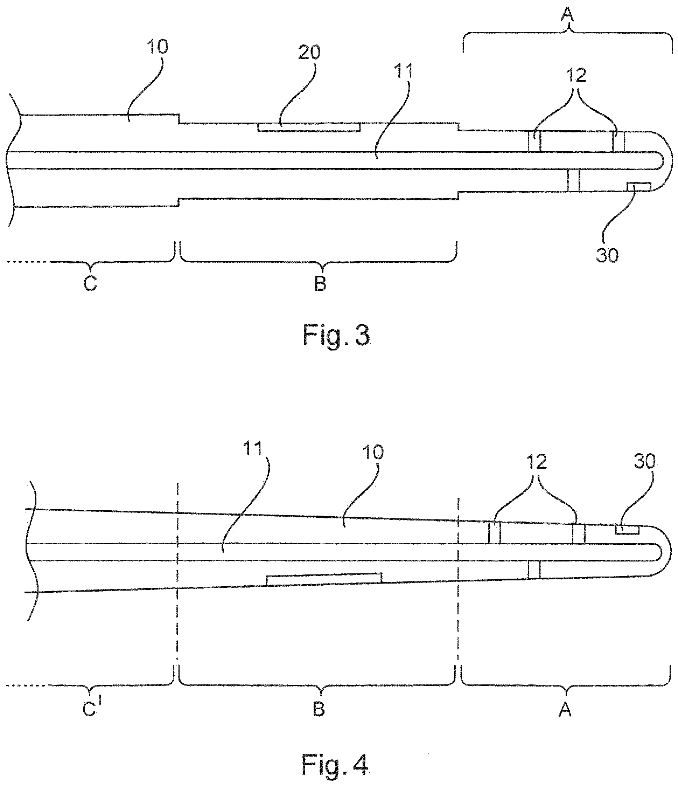

Die Figuren 3 und 4 beschreiben beispielhafte Ausführungsformen bezüglich der äußeren Form der Gehirndrainagevorrichtung 1 bzw. des stabförmigen Hohlkörpers 10. Figur 3 zeigt dabei eine Ausgestaltung, bei der der stabförmige Hohlkörper eine gestufte Außengeometrie aufweist, die beispielsweise an den Stellen gestuft sein kann, in denen der voraussichtliche Übergang zwischen der Schädeldecke 2 und der Gehirnmasse 4 sowie zwischen der Gehirnmasse 4 und dem Ventrikel 5 erwartet wird. Mit anderen Worten können die Stufungen beispielsweise zwischen dem Bereich A und dem Bereich B und/oder zwischen dem Bereich B und Bereich C erfolgen. Wie in Bezugnahme auf Figur 1 bereits beschrieben wurde, liegen dann im Bereich B wiederum die erste Sensorvorrichtung 20 und im Bereich A die zweite Sensorvorrichtung 30, wenn diese vorgesehen werden soll. Die Drainageöffnungen 12 liegen hier im Bereich A. Für den Fall, dass eine solche abgesetzte Außengeometrie des stabförmigen Hohlkörpers 10 vorgesehen ist, insbesondere zwischen den Bereichen B und C, kann dies die Festlegung an der äußeren Schädeldecke erleichtern und verhindern, dass der stabförmige Hohlkörper weiter in den Schädel eindringt. Da die Anatomie der Patienten variieren kann, können unterschiedliche Sondenlängen vorkonfektioniert bereitgehalten werden oder auch individualisiert vorgefertigt werden. Es sei verstanden, dass ein derartiger Absatz auch ausschließlich zwischen den Bereichen B und C vorgesehen sein kann, wobei in diesem Fall dann der Übergang zwischen den Bereichen A und B bezüglich der Außengeometrie im Wesentlichen kontinuierlich verläuft. Figur 4 zeigt eine weitere Ausgestaltung, bei der die äußere Geometrie des stabförmigen Hohlkörpers C konisch ausgestaltet ist, sodass an dieser Stelle keine äußeren Stufen vorgesehen sind. Die Lage der ersten und zweiten Sensoranordnung 20, 30 ist analog zu Figur 3, ebenso wie die Lage der Drainageöffnungen 12. Es sei verstanden, dass auch eine Mischform einer Geometrie verwendet werden kann, bei der beispielsweise zwischen den Bereichen B und C eine Stufe vorgesehen ist, während der Bereich B und A im Wesentlichen konisch oder zylindrisch verläuft.

Figur 5 zeigt eine weitere beispielhafte Ausführungsform des stabförmigen Hohlkörpers 10 mit dem darin liegenden inneren Drainagekanal 11. In der in Figur 5 gezeigten Ausführungsform weist der stabförmige Hohlkörper im Bereich der Stirnfläche keine Drainageöffnung auf, sodass eine derartige Ausgestaltung möglicherweise weniger Verletzungen hervorruft. Die im zylindrischen Bereich des Bereiches A vorgesehene Drainageöffnung 12 kann beispielsweise rund, oval oder elliptisch ausgestaltet sein. Eine Ausgestaltung des Bereichs A mit nur einer einzigen stirnseitigen Drainageöffnung 12 ermöglicht den Bereich A kurz zu halten, um auch in sehr flachen Ventrikelbereichen zu drainagieren. Die Mündung der Drainageöffnung 12 kann dabei gerundete Ecken an der Außenseite des stabförmigen Hohlkörpers aufweisen, sodass Abscherungen von Gewebe beim Einführen im Wesentlichen vermieden werden kann. In diesem Bereich A wiederum können auch Sensoren vorgesehen sein, in der hier gezeigten Ausführungsform beispielsweise ein Drucksensor oder ein Temperatursensor. Diese Sensoren können beispielsweise an der zylindrischen Außenwand des Bereiches A vorgesehen sein oder auch im Stirnbereich.

Figur 6 zeigt eine Teilansicht einer Schnittansicht der Gehirndrainagevorrichtung 1 bei der mehrere gleichförmige Sensoren entlang der Längserstreckung vorgesehen sein können. In diesem Fall sind im Bereich B die Sensoren 23 bzw. 24 mehrfach vorgesehen, sodass eine ortsaufgelöste Messung erfolgen kann. Eine derartige ortsaufgelöste Messung kann beispielsweise eine Temperaturmessung, eine Sauerstoffsättigungsmessung oder eine Gehirnaktivitätsmessung sein. Es sei verstanden, dass derartige Mehrfachsensoren auch im Bereich A vorgesehen sein können, der im Ventrikelbereich des Gehirns liegt.

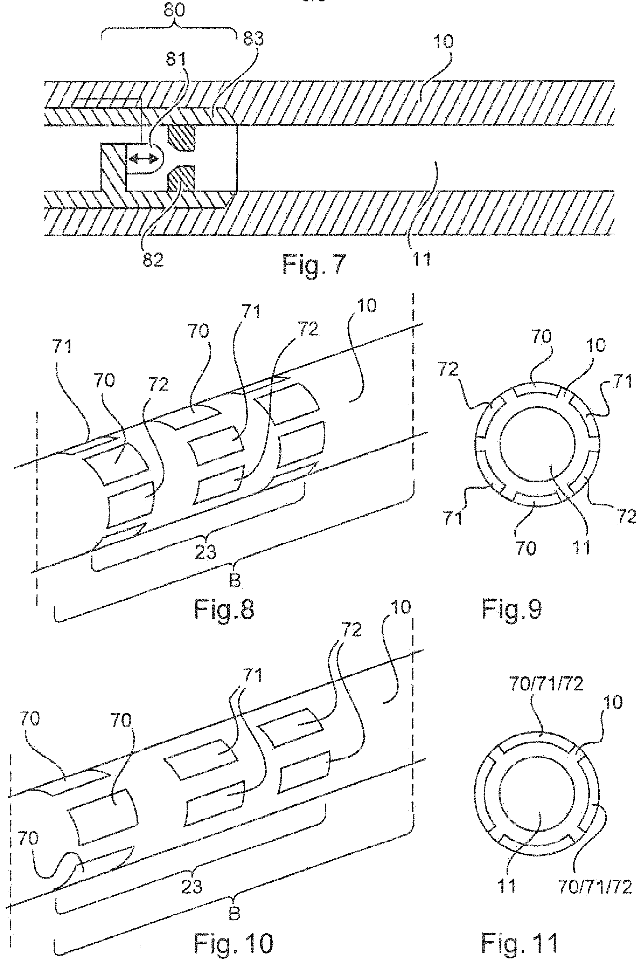

Figur 7 zeigt eine Schnittansicht einer weiteren beispielhaften Ausführungsform der Erfindung, bei der im Inneren des stabförmigen Hohlkörpers 10 eine Ventilanordnung 80 vorgesehen ist. Diese Ventilanordnung kann dazu dienen, den Drainagefluss durch den Drainagekanal 11 zu steuern. Zu diesem Zweck weist die Ventilanordnung beispielsweise einen Ventilsitz 82 sowie ein bewegliches Ventilelement 81 auf. Der Ventilsitz 82 kann dabei beispielsweise fest mit einer Wandung 83 des Drainagekanals 11 verbunden sein. Das Ventilelement 81 kann sich dabei in Bezug auf die Position der Innenwand des Drainagekanals 11 bewegen, um dichtend in Eingriff mit dem Ventilsitz 82 zu gelangen. Das Ventilelement kann dabei angesteuert werden, sodass sich beispielsweise ein konstanter voreinstellbarer Druck einstellt und das Ventil diesen Druck im Wesentlichen geregelt aufrechterhält. In der in Figur 7 gezeigten Ausführungsform ist die Innenwandung 83 des Drainagerohrs als ein separates Rohr vorgesehen, wobei dieses Rohr 83 als eine Basis für ein Ventilmodul dienen kann, welches vorgefertigt werden kann. Dabei kann an einem derartigen Rohr 83 der Ventilsitz 82 befestigt sein, sowie das Ventilelement 81 mit entsprechenden Steuerleitungen. Ein derartiges Modul, bestehend aus den Elementen Innenrohr 83, Ventilsitz 82 und Ventilelement 81 kann dabei als vorgefertigtes Element in den inneren Drainagekanal des stabförmigen Hohlkörpers 10 bei der Fertigung eingeführt werden. Zu diesem Zweck kann die Innenwandung des Drainagekanals 11 einen Absatz bzw. einen Rücksprung aufweisen, sodass die Innenwandung des Rohres 83 zusammen mit der verbleibenden Innenwandung des Drainagekanals 11 eine fluchtende Fläche bildet und keine Absätze oder Lücken bildet, die bezüglich einer Sterilisierung möglicherweise zu Problemen führen könnten. Es sei verstanden, dass eine derartige Ventilvorrichtung proximal zu den Drainageöffnungen vorgesehen sein kann, und sowohl im Bereich C als auch im Bereich B oder A angeordnet sein kann.

Figur 8 zeigt eine beispielhafte Ausführungsform der Außenseite des stabförmigen Hohlkörpers 10, bei dem hier am Beispiel eines Sensors 23 der strukturelle Aufbau erläutert wird. In der in Figur 8 gezeigten Anordnung weist der Sauerstoffsensor eine oder mehrere Lichtquellen 70 auf, die Licht mit unterschiedlichen Wellenlängen emittieren kann/können. Das emittierte Licht kann dabei beispielsweise im Rotbereich oder im Infrarotbereich liegen. Bevorzugt sind hier beispielsweise Wellenlängen oberhalb von 570 nm bzw. oberhalb von 600 nm. Korrespondierend dazu sind entsprechende lichtempfindliche Sensoren 71 und 72 vorgesehen, die in der in Figur 8 gezeigten Anordnung auf der gleichen Höhe neben der Lichtquelle 70 angeordnet sind. Der erste lichtempfindliche Sensor 71 ist dabei ein Lichtsensor, der empfindlich auf eine erste Wellenlänge der Lichtquelle 70 reagiert, während der zweite Lichtsensor 72 ein Sensor ist, der auf eine zweite davon verschiedene Wellenlänge reagiert. Auf diese Weise können aufgrund unterschiedlicher Wellenlänge unterschiedliche Parameter bei der Sauerstoffsättigung gemessen werden. Es sei verstanden, dass mehrere derartige Anordnungen bestehend aus einer Lichtquelle 70 und zwei Lichtsensoren 71 und 72 vorgesehen sein können, wie in Figur 8 gezeigt. Dabei sei verstanden, dass eine derartige Sensoranordnung nicht beschränkt ist auf die in Figur 8 gezeigten drei Ringe. Die zwei Lichtsensoren 71 und 72 können auch in einem einzigen Bauelement realisiert sein.

Korrespondierend dazu zeigt Figur 9 eine Schnittansicht durch den stabförmigen Hohlkörper 10 mit dem darin liegenden Drainagekanal 11. Entlang des Umfangs sind hier wiederum die Lichtquelle 70 sowie der erste lichtempfindliche Sensor 71 und der zweite lichtempfindliche Sensor 72 angeordnet. In der in Figur 9 gezeigten Anordnung sind entlang des Umfangs zwei Lichtquellen, zwei erste lichtempfindliche Sensoren und zwei zweite lichtempfindliche Sensoren vorgesehen, die so im Wechsel angeordnet sind, dass neben der Lichtquelle 70 jeweils ein erster und ein zweiter lichtempfindlicher Sensor 71, 72 liegt. Es sei verstanden, dass eine derartige Anordnung auch beispielsweise Lichtquelle 70 und jeweils einen ersten lichtempfindlichen Sensor 71 und einen lichtempfindlichen Sensor 72 entlang des Umfangs aufweisen kann.

Figur 10 zeigt eine weitere Ausgestaltung der Erfindung, bei der einer oder mehrere Lichtquellen 70 auf einer Höhe angeordnet sind, während die lichtempfindlichen Sensoren 71 und 72 davon in Längsrichtung beabstandet angeordnet sind. Es sei dabei verstanden, dass auch mehrere erste Sensoren und zweite Sensoren entlang des Umfangs verteilt sein können und dass auch die Lichtquelle 70 in der Mitte zwischen dem ersten lichtempfindlichen Sensor und dem zweiten lichtempfindlichen Sensor 71, 72 angeordnet sein können.

Figur 11 zeigt eine zu Figur 10 korrespondierende Schnittansicht des stabförmigen Hohlkörpers 10 mit dem darin liegenden inneren Drainagekanal 11, bei der in einer Schnittebene durch die Sensoren jeweils nur ein Typ von Sensor bzw. die Lichtquelle angeordnet sind.

Die in den Figuren 8 bis 11 gezeigten Sensoren können dabei in einer in der Außenwand des stabförmigen Hohlkörpers 10 vorgesehenen Ausnehmung oder einer Mehrzahl von Ausnehmungen 13 eingebettet sein, sodass diese Sensoren und Lichtquellen nicht über die Außenfläche des verbleibenden stabförmigen Hohlkörpers hinausragen. Die Lichtquelle 70 kann dabei von dem Messsystem gesteuert werden und beispielsweise dauerhaft angesteuert werden oder gepulst betrieben werden. Es sei verstanden, dass die Sensoren zur Erfassung des Lichtes mit unterschiedlichen Wellenlängen auch mehrere unterschiedliche Wellenlängen erfassen können und eine Mehrzahl derartiger Sensoren auch ein entsprechendes Erfassungsarray bilden können, sodass aufgrund der örtlichen Verteilung und der dort ermittelten Lichtintensitäten mit entsprechenden Wellenlängen auf beispielsweise eine Sauerstoffsättigung zurückgeschlossen werden kann. Insbesondere können punktförmige Lichtquellen und auch im Wesentlichen punktförmige Sensoren verwendet werden sowie auch sektorielle Lichtquellen und Sensoren.

Darüber hinaus können auch in analoger Geometrie zu den Figuren 8 und 10 sowie 9 und 11 Sensoren zur Erfassung der Gehirnaktivität vorgesehen sein. Derartige Sensoren können dabei den Potentialunterschied unter einander, d.h. zwischen unterschiedlichen Sensoren, als auch gegenüber einem externen Bezugspotential erfassen. Die Elektroden können dabei gleich große Flächen aufweisen, jedoch auch unterschiedlich große Flächen und Geometrie aufweisen, je nach gewünschter Anwendungsart. Es sei verstanden, dass derartige Elektroden und Sensoren ebenfalls drucktechnisch hergestellt werden können gemäß den oben erwähnten drucktechnischen Verfahren. Das Vorsehen von derartigen Elektroden zur Messung der Gehirnaktivität hat gegenüber den Elektroden, die üblicherweise auf die Außenseite des Schädels aufgesetzt werden, den entscheidenden Vorteil, dass diese unmittelbar am Ort der Gehirnaktivitätsentwicklung messen und keine Signalverluste oder Verzerrungen über die Schädeldecke aufweisen. Auch eine derartige Elektrodenanordnung zur Erfassung der Gehirnaktivität wird üblicherweise im Bereich B des stabförmigen Hohlkörpers vorgesehen, der im Bereich der Gehirnmasse 4 liegt.

Ergänzend ist darauf hinzuweisen, dass "umfassend" keine anderen Elemente oder Schritte ausschließt und "eine" oder "ein" keine Vielzahl ausschließt. Ferner sei darauf hingewiesen, dass Merkmale, die mit Verweis auf eines der obigen Ausführungsbeispiele beschrieben worden sind, auch in Kombination mit anderen Merkmalen anderer oben beschriebener Ausführungsbeispiele verwendet werden können. Bezugszeichen in den Ansprüchen sind nicht als Einschränkung anzusehen.

### Bezugszeichenliste:

- A: erster Bereich/Vertrikelbereich
- B: zweiter Bereich/Gehirnmassebereich
- C: dritter Bereich/Schädeldeckenbereich
- 1: Gehirndrainagevorrichtung
- 2: Schädeldecke
- 3: Gehirn
- 4: Gehirnmasse
- 5: Ventrikel
- 9: Messsystem
- 10: stabförmigen Hohlkörper
- 11: innerer Drainagekanal des stabförmigen Hohlkörpers
- 12: Drainageöffnung/Drainageöffnungsanordnung
- 13: Vertiefung/Ausnehmung zur Aufnahme eines Sensors/Sensoranordnung
- 20: erste Sensoranordnung
- 21: Sensor/Temperatursensor der ersten Sensoranordnung
- 22: Sensor/Drucksensor der ersten Sensoranordnung
- 23: Sensor/Sauerstoffsättigungssensor der ersten Sensoranordnung
- 24: Sensor/Gehirnaktivitätssensor der ersten Sensoranordnung
- 30: zweite Sensoranordnung
- 31: Sensor/Temperatursensor der zweiten Sensoranordnung
- 32: Sensor/Drucksensor der zweiten Sensoranordnung
- 33: Sensor/Sauerstoffsättigungssensor der zweiten Sensoranordnung
- 40: Signalverbindung
- 50: Signalschnittstelle
- 61: Cranialblock
- 62: Kontermutter zum Cranialblock
- 63: Drainageleitung
- 64: Signal-/Datenleitung
- 70: Lichtquelle des Sauerstoffsättigungssensors
- 71: erster Lichtsensor des Sauerstoffsättigungssensors
- 72: zweiter Lichtsensor des Sauerstoffsättigungssensors
- 80: steuerbare Ventilanordnung
- 81: Ventilelement
- 82: Ventilsitz
- 83: Ventilabschnitt der Innenwandung des Drainagekanals

## Patentansprüche

1. Gehirndrainagevorrichtung mit
einem stabförmigen Hohlkörper (10) mit einem inneren Drainagekanal (11) zur Einführung durch die Schädeldecke (2) in das Gehirn (3),
einer ersten Sensoranordnung (20) mit wenigsten einem Sensor (21, 22, 23, 24) zur Messung eines physikalischen Parameters und
einer Signalschnittstelle (50);
wobei der stabförmige Hohlkörper (10) einen ersten Bereich (A) aufweist, der ausgelegt ist, um im applizierten Zustand in einen im Gehirn befindlichen Ventrikel (5) hineinzuragen;
wobei der stabförmige Hohlkörper (10) einen zweiten Bereich (B) aufweist, der proximal von dem ersten Bereich (A) angeordnet ist, wobei der zweite Bereich (B) ausgelegt ist, um im applizierten Zustand im Bereich der Gehirnmasse (4) zu liegen;
wobei der erste Bereich (A) eine mit dem Drainagekanal (11) verbundene Drainageöffnungsanordnung (12) aufweist, über die im applizierten Zustand Liquor aus dem Ventrikel (5) in den Drainagekanal (11) drainagiert werden kann;
wobei die erste Sensoranordnung (20) im zweiten Bereich (B) angeordnet ist, um einen physikalischen Parameter der Gehirnmasse (4) zu messen;
wobei die erste Sensoranordnung (20) mit der Signalschnittstelle (50) verbunden ist, um von der ersten Sensoranordnung (20) ermittelte Messdaten an ein zu verbindendes Messsystem (9) zu übermitteln,
wobei die erste Sensoranordnung (20) wenigstens einen Sensor zur Erfassung eines Sauerstoffgehaltes und/oder einer Sauerstoffsättigung im Bereich der Gehirnmasse (4) oder des Ventrikels (5) aufweist, wobei der Sensor wenigstens eine Lichtquelle (70) für Licht mit wenigstens einer ersten Wellenlänge und einer zweiten Wellenlänge aufweist, sowie wenigstens einen der Lichtquelle (70) zugeordneten und bezüglich der erste Wellenlänge sensitiven ersten Lichtsensor (71) und einen der Lichtquelle (70) zugeordneten und bezüglich der zweite Wellenlänge sensitiven zweiten Lichtsensor (72).

2. Gehirndrainagevorrichtung gemäß Anspruch 1, wobei die erste Sensoranordnung (20) ferner wenigstens einen Sensor (21, 22, 23, 24) aufweist aus der Gruppe bestehend aus: Drucksensor, Temperatursensor und Gehirnaktivitätssensor.

3. Gehirndrainagevorrichtung gemäß einem der Ansprüche 1 und 2, wobei der erste, mit der Drainageöffnungsanordnung (12) versehene Bereich (A) eine zweite Sensoranordnung (30) aufweist mit wenigstens einem Sensor (31, 32, 33) aus der Gruppe bestehend aus: Sauerstoffgehaltsensor, Drucksensor und Temperatursensor, um einen entsprechenden physikalischen Parameter im Ventrikel (5) zu ermitteln, wobei die zweite Sensoranordnung (30) mit der Signalschnittstelle (50) verbunden ist, um von der zweiten Sensoranordnung (30) ermittelte Messdaten an ein zu verbindendes Messsystem (9) zu übermitteln.

4. Gehirndrainagevorrichtung gemäß Anspruch 3, wobei wenigstens ein Sensor (21, 31) der zweiten Sensoranordnung (20, 30) ein Sensor zur Erfassung eines Sauerstoffgehaltes und/oder einer Sauerstoffsättigung im Bereich der Gehirnmasse (4) oder des Ventrikels (5) ist und wenigstens eine Lichtquelle (70) für Licht mit wenigstens einer ersten Wellenlänge und einer zweiten Wellenlänge aufweist, sowie wenigstens einen der Lichtquelle (70) zugeordneten und bezüglich der erste Wellenlänge sensitiven ersten Lichtsensor (71) und einen der Lichtquelle (70) zugeordneten und bezüglich der zweite Wellenlänge sensitiven zweiten Lichtsensor (72).

5. Gehirndrainagevorrichtung gemäß einem der Ansprüche 1 bis 4, wobei der erste Lichtsensor (71) und der zweite Lichtsensor (72) axial und/oder azimutal beabstandet zu der zugeordneten Lichtquelle (70) angeordnet sind.

6. Gehirndrainagevorrichtung gemäß einem der Ansprüche 1 bis 5, wobei die Drainageöffnungsanordnung (12) wenigstens eine ovale oder elliptische Öffnung aufweist.

7. Gehirndrainagevorrichtung gemäß einem der Ansprüche 1 bis 6, wobei die Gehirndrainagevorrichtung im Drainagekanal (11) proximal der Drainageöffnungsanordnung (12) eine steuerbare Ventilanordnung (80) aufweist, um den Drainagekanal (11) gezielt zu öffnen und zu schließen in Abhängigkeit von einem durch eine der Sensoranordnungen (20, 30) gemessenen Druck.

8. Gehirndrainagevorrichtung gemäß Anspruch 7, wobei die steuerbare Ventilanordnung (80) einen mit einer Innenwandung des Drainagekanals (11) verbundenen Ventilsitz (82) und ein dazu in Bezug zu dem Drainagekanal (11) verschiebbares und gegenüber dem Ventilsitz (82) abdichtendes Ventilelement (81) aufweist.

9. Gehirndrainagevorrichtung gemäß Anspruch 8, wobei ein Abschnitt (83) der Innenwandung des Drainagekanals (11), der Ventilsitz (82) und das Ventilelement (81) als eine vormontierte Einheit (80) ausgebildet sind, die bei der Fertigung in den stabförmigen Hohlkörper (10) eingebracht werden kann.

10. Gehirndrainagevorrichtung gemäß einem der Ansprüche 1 bis 9, wobei die Gehirndrainagevorrichtung (1) ferner einen dritten Bereich (C) aufweist, der proximal zum zweiten Bereich (B) angeordnet ist, wobei der dritte Bereich (C) ausgelegt ist die Gehirndrainagevorrichtung (1) an der Schädeldecke (2) festzulegen.

11. Gehirndrainagevorrichtung gemäß einem der Ansprüche 1 bis 10, wobei die erste Sensoranordnung (20) wenigstens einen der Sensoren (21, 22, 23, 24) zur Messung der Temperatur, des Gehirndruckes, des Sauerstoffgehaltes bzw. der Gehirnaktivität mehrfach aufweist, wobei die Mehrzahl der Sensoren einer Sensorart entlang einer Längserstreckungsrichtung des stabförmigen Hohlkörpers (10) angeordnet ist, um eine ortsaufgelöste Messung eines entsprechenden physikalischen Parameters vornehmen zu können.

12. Gehirndrainagevorrichtung gemäß einem der Ansprüche 1 bis 11, wobei der stabförmige Hohlkörper (10) wenigstens eine Ausnehmung (13) im ersten Bereich (A) und/oder im zweiten Bereich (B) aufweist, wobei die erste bzw. zweite Sensoranordnung (20, 30) in der Ausnehmung (13) außenflächenbündig angeordnet ist.

13. Gehirndrainagevorrichtung gemäß einem der Ansprüche 1 bis 12, wobei eine Signalverbindung (40) zwischen der ersten Sensoranordnung (20) bzw. der zweiten Sensoranordnung (30) und der Signalschnittstelle (50) eine drucktechnisch hergestellte Signalverbindung ist.

14. Gehirndrainagevorrichtung gemäß einem der Ansprüche 1 bis 13, wobei wenigstens einer der Sensoren (21, 22, 23, 24) der ersten Sensoranordnung (20) und/oder der zweiten Sensoranordnung (31, 32, 33) ein drucktechnisch hergestellter Sensor ist.

15. Gehirndrainagevorrichtung gemäß einem der Ansprüche 1 bis 14, wobei der stabförmige Hohlkörper (10) aus einem magnetisch inaktivem Material gefertigt ist.

## Claims

1. A brain drainage device with
a rod-shaped hollow body (10) with an inner drainage channel (11) for insertion through the cranium (2) into the brain (3),
a first sensor arrangement (20) with at least one sensor (21, 22, 23, 24) for measuring a physical parameter, and
a signal interface (50);
wherein the rod-shaped hollow body (10) has a first region (A) that, in the applied state, is designed to protrude into a ventricle (5) situated in the brain;
wherein the rod-shaped hollow body (10) has a second region (B) that is arranged proximally from the first region (A), wherein the second region (B) is designed to lie in the region of the cerebral mass (4) in the applied state;
wherein the first region (A) has a drainage opening arrangement (12) connected to the drainage channel (11) through which liquor can be drained from the ventricle (5) into the drainage channel (11) in the applied state;
wherein the first sensor arrangement (20) is arranged in the second region (B) to measure a physical parameter of the cerebral mass (4);
wherein the first sensor arrangement (20) is connected to the signal interface (50) to transmit measurement data determined by the first sensor arrangement (20) to a measuring system (9) that is to be connected,
wherein the first sensor arrangement (20) has at least one sensor for detecting an oxygen content and/or an oxygen saturation in the region of the cerebral mass (4) or the ventricle (5), wherein the sensor has at least one light source (70) for light with at least a first wavelength and a second wavelength, as well as at least one first light sensor (71) associated with the light source (70) and sensitive to the first wavelength and a second light sensor (72) associated with the light source (70) and sensitive to the second wavelength.

2. Brain drainage device according to claim 1, wherein the first sensor arrangement (20) further has at least one sensor (21, 22, 23, 24) from the group consisting of: pressure sensor, temperature sensor, and brain activity sensor.

3. Brain drainage device according to any one of claims 1 and 2, wherein the first region (A) provided with the drainage opening arrangement (12) has a second sensor arrangement (30) with at least one sensor (31, 32, 33) from the group consisting of: oxygen content sensor , pressure sensor and temperature sensor to determine a corresponding physical parameter in the ventricle (5), wherein the second sensor arrangement (30) is connected to the signal interface (50) to transmit measurement data determined by the second sensor arrangement (30) to a measuring system (9) that is to be connected.

4. Brain drainage device according to claim 3, wherein at least one sensor (21, 31) of the second sensor arrangement (20, 30) is a sensor for detecting an oxygen content and/or an oxygen saturation in the region of the cerebral mass (4) or the ventricle (5) and has at least one light source (70) for light with at least a first wavelength and a second wavelength, as well as at least one first light sensor (71) associated with the light source (70) and sensitive to the first wavelength and a second light sensor (72) associated with the light source (70) and sensitive to the second wavelength.

5. Brain drainage device according to any one of claims 1 to 4, wherein the first light sensor (71) and the second light sensor (72) are arranged axially and/or azimuthally spaced from the associated light source (70).

6. Brain drainage device according to any one of claims 1 to 5, wherein the drainage opening arrangement (12) has at least one oval or elliptical opening.

7. Brain drainage device according to any one of claims 1 to 6, wherein the brain drainage device in the drainage channel (11) proximal to the drainage opening arrangement (12) has a controllable valve arrangement (80) to selectively open and close the drainage channel (11) in response to a pressure measured by one of the sensor arrangements (20, 30).

8. Brain drainage device according to claim 7, wherein the controllable valve arrangement (80) has a valve seat (82) connected to an inner wall of the drainage channel (11) and a valve element (81) that is displaceable with respect to the drainage channel (11) and that is sealing relative to the valve seat (82).

9. Brain drainage device according to claim 8, wherein a portion (83) of the inner wall of the drainage channel (11), the valve seat (82) and the valve element (81) are formed as a pre-assembled unit (80) that can be introduced into the rod-shaped hollow body (10) during manufacture.

10. Brain drainage device according to any one of claims 1 to 9, wherein the brain drainage device (1) further has a third region (C) that is arranged proximal to the second region (B), wherein the third region (C) is designed to attach the brain drainage device (1) to the cranium (2).

11. Brain drainage device according to any one of claims 1 to 10, wherein the first sensor arrangement (20) has at least one of the sensors (21, 22, 23, 24) for measuring the temperature, the brain pressure, the oxygen content or the brain activity multiple times, wherein the plurality of sensors of a sensor type are arranged along a longitudinal extension direction of the rod-shaped hollow body (10) to be able to perform a spatially resolved measurement of a corresponding physical parameter.

12. Brain drainage device according to any one of claims 1 to 11, wherein the rod-shaped hollow body (10) has at least one recess (13) in the first region (A) and/or in the second region (B), wherein the first or second sensor arrangement (20, 30) is arranged flush with the surface in the recess (13).

13. Brain drainage device according to any one of claims 1 to 12, wherein a signal connection (40) between the first sensor arrangement (20) or the second sensor arrangement (30) and the signal interface (50) is a signal connection produced by printing technology.

14. Brain drainage device according to any one of claims 1 to 13, wherein at least one of the sensors (21, 22, 23, 24) of the first sensor arrangement (20) and/or the second sensor arrangement (31, 32, 33) is a sensor produced by printing technology.

15. Brain drainage device according to any one of claims 1 to 14, wherein the rod-shaped hollow body (10) is made of a magnetically inactive material.

## Revendications

1. Dispositif de drainage cérébral comportant :
un corps creux en forme de tige (10) avec un canal de drainage interne (11) destiné à être inséré à travers la calotte crânienne (2) dans le cerveau (3),
un premier ensemble de capteurs (20) comportant au moins un capteur (21, 22, 23, 24) pour mesurer un paramètre physique et
une interface de signaux (50) ;
dans lequel le corps creux en forme de tige (10) comporte une première zone (A) conçue pour faire saillie, à l'état appliqué, dans un ventricule (5) situé dans le cerveau ;
dans lequel le corps creux en forme de tige (10) comporte une deuxième zone (B) disposée de manière proximale à la première zone (A), dans lequel la deuxième zone (B) est conçue pour être, à l'état appliqué, dans la zone de la masse cérébrale (4) ;
dans lequel la première zone (A) comporte un ensemble d'orifices de drainage (12) relié au canal de drainage (11), par l'intermédiaire duquel, à l'état appliqué, du liquide peut être drainé à partir du ventricule (5) dans le canal de drainage (11) ;
dans lequel le premier ensemble de capteurs (20) est disposé dans la deuxième zone (B) afin de mesurer un paramètre physique de la masse cérébrale (4) ;
dans lequel le premier ensemble de capteurs (20) est relié à l'interface de signaux (50) afin de transmettre des données de mesure déterminées par le premier ensemble de capteurs (20) à un système de mesure (9) à raccorder,
dans lequel le premier ensemble de capteurs (20) comporte au moins un capteur pour détecter une teneur en oxygène et/ou une saturation en oxygène dans la zone de la masse cérébrale (4) ou du ventricule (5), dans lequel le capteur comporte au moins une source lumineuse (70) pour de la lumière ayant au moins une première longueur d'onde et une seconde longueur d'onde, ainsi qu'au moins un premier capteur de lumière (71) associé à la source lumineuse (70) et sensible à la première longueur d'onde et un second capteur de lumière (72) associé à la source lumineuse (70) et sensible à la seconde longueur d'onde.

2. Dispositif de drainage cérébral selon la revendication 1, dans lequel le premier ensemble de capteurs (20) comporte en outre au moins un capteur (21, 22, 23, 24) du groupe constitué d'un capteur de pression, d'un capteur de température et d'un capteur d'activité cérébrale.

3. Dispositif de drainage cérébral selon l'une des revendications 1 et 2, dans lequel la première zone (A) pourvue de l'ensemble d'orifices de drainage (12) comporte un second ensemble de capteurs (30) avec au moins un capteur (31, 32, 33) du groupe constitué d'un capteur de teneur en oxygène, d'un capteur de pression et d'un capteur de température, afin de déterminer un paramètre physique correspondant dans le ventricule (5), dans lequel le second ensemble de capteurs (30) est relié à l'interface de signaux (50) afin de transmettre des données de mesure déterminées par le second ensemble de capteurs (30) à un système de mesure (9) à raccorder.

4. Dispositif de drainage cérébral selon la revendication 3, dans lequel au moins un capteur (21, 31) du second ensemble de capteurs (20, 30) est un capteur destiné à détecter une teneur en oxygène et/ou une saturation en oxygène dans la zone de la masse cérébrale (4) ou du ventricule (5) et comporte au moins une source lumineuse (70) pour de la lumière ayant au moins une première longueur d'onde et une seconde longueur d'onde, ainsi qu'au moins un premier capteur de lumière (71) associé à la source lumineuse (70) et sensible à la première longueur d'onde et un second capteur de lumière (72) associé à la source lumineuse (70) et sensible à la seconde longueur d'onde.

5. Dispositif de drainage cérébral selon l'une des revendications 1 à 4, dans lequel le premier capteur de lumière (71) et le second capteur de lumière (72) sont disposés espacés de manière axiale et/ou azimutale par rapport à la source lumineuse (70) associée.

6. Dispositif de drainage cérébral selon l'une des revendications 1 à 5, dans lequel l'ensemble d'orifices de drainage (12) comporte au moins un orifice ovale ou elliptique.

7. Dispositif de drainage cérébral selon l'une des revendications 1 à 6, dans lequel le dispositif de drainage cérébral comporte, dans le canal de drainage (11) proximal à l'ensemble d'orifices de drainage (12), un ensemble de soupape (80) pouvant être commandé pour ouvrir et fermer sélectivement le canal de drainage (11) en fonction d'une pression mesurée par l'un des ensembles de capteur (20, 30) .

8. Dispositif de drainage cérébral selon la revendication 7, dans lequel l'ensemble de soupape (80) pouvant être commandé comporte un siège de soupape (82) relié à une paroi interne du canal de drainage (11) et comporte un élément de soupape (81) qui peut déplacé par rapport au canal de drainage (11) et hermétique par rapport au siège de soupape (82).

9. Dispositif de drainage cérébral selon la revendication 8, dans lequel une partie (83) de la paroi interne du canal de drainage (11), le siège de soupape (82) et l'élément de soupape (81) sont réalisés sous la forme d'une unité prémontée (80) qui peut être introduite dans le corps creux en forme de tige (10) lors de la fabrication.

10. Dispositif de drainage cérébral selon l'une des revendications 1 à 9, dans lequel le dispositif de drainage cérébral (1) comporte en outre une troisième zone (C) disposée de manière proximale à la deuxième zone (B), dans lequel la troisième zone (C) est conçue pour fixer le dispositif de drainage cérébral (1) sur la calotte crânienne (2).

11. Dispositif de drainage cérébral selon l'une des revendications 1 à 10, dans lequel le premier ensemble de capteurs (20) comporte au moins l'un des capteurs (21, 22, 23, 24) pour mesurer plusieurs fois la température, la pression cérébrale, la teneur en oxygène ou l'activité cérébrale, dans lequel la pluralité des capteurs d'un type de capteur est disposée le long d'une direction d'extension longitudinale du corps creux en forme de tige (10) afin de pouvoir effectuer une mesure spatialement résolue d'un paramètre physique correspondant.

12. Dispositif de drainage cérébral selon l'une des revendications 1 à 11, dans lequel le corps creux en forme de tige (10) comporte au moins un évidement (13) dans la première zone (A) et/ou dans la deuxième zone (B), dans lequel le premier ou second ensemble de capteurs (20, 30) est disposé dans l'évidement (13) de manière à affleurer la surface extérieure.

13. Dispositif de drainage cérébral selon l'une des revendications 1 à 12, dans lequel une liaison par signaux (40) entre le premier ensemble de capteurs (20) ou le second ensemble de capteurs (30) et l'interface de signaux (50) est une liaison par signaux fabriquée par une technique d'impression.

14. Dispositif de drainage cérébral selon l'une des revendications 1 à 13, dans lequel au moins l'un des capteurs (21, 22, 23, 24) du premier ensemble de capteurs (20) et/ou du second ensemble de capteurs (31, 32, 33) est un capteur fabriqué par une technique d'impression.

15. Dispositif de drainage cérébral selon l'une des revendications 1 à 14, dans lequel le corps creux en forme de tige (10) est fabriqué à partir d'un matériau magnétiquement inactif.
